(19) Europäisches Patentamt
European Patent Office
Office européen des brevets

(11) Publication number: 0 488 224 A2

(12) **EUROPEAN PATENT APPLICATION**

(21) Application number: **91120285.1**

(22) Date of filing: **27.11.91**

(51) Int. Cl.$^5$: **C12P 21/00**, C12N 1/14, //A23K1/17,(C12P21/00, C12R1:645)

(30) Priority: **28.11.90 JP 328424/90**
**07.03.91 JP 67988/91**

(43) Date of publication of application:
**03.06.92 Bulletin 92/23**

(84) Designated Contracting States:
**DE DK FR GB NL**

(71) Applicant: **MITSUBISHI KASEI CORPORATION**
**5-2, Marunouchi 2-chome Chiyoda-ku**
**Tokyo 100(JP)**

(72) Inventor: **Hino, Tsuneo**
**F5-204, Green-haitsu, 571, Unomori**
**Sagamihara-shi, Kanagawa-ken(JP)**
Inventor: **Kanda, Mina**
**201, Sharumu-Shibuya, 2-7-18, Morino**
**Machida-shi, Tokyo-to(JP)**
Inventor: **Kumazawa, Shigenori**
**12-306, Machida-cooptown, 2-10-2, Ogawa**
**Machida-shi, Tokyo-to(JP)**
Inventor: **Yoshikawa, Nobuji**
**8-404, Popuragaoka-coop, 2-11-2, Naruse**
**Machida-shi, Tokyo-to(JP)**
Inventor: **Mikawa, Takashi**
**1-17-4-3, Nishihashimoto**
**Sagamihara-shi, Kanagawa-ken(JP)**

(74) Representative: **Hansen, Bernd, Dr.**
**Dipl.-Chem. et al**
**Hoffmann, Eitle & Partner Patent- und**
**Rechtsanwälte Arabellastrasse 4 Postfach**
**81 04 20**
**W-8000 München 81(DE)**

(54) **Fermentationproduct of a Verticimonosporium strain and feed additivs containing a linear peptide.**

(57) A novel peptide compound, substance MBA090-18, which is produced by Verticimonosporium ellipticum D1528 (FERM BP-3663) and has anti-parasite, anti-nematode and ionophore activities, an anthelmintic agent, which comprises as an active ingredient said substance MBA090-18, and a feed additive useful for improving the feed effeciency of animal, which comprises as an active ingredient a linear peptide compound including the substance MBA090-18.

EP 0 488 224 A2

This invention relates to novel peptide compounds. More particularly, it relates to a novel peptide compound named substance MBA090-18 having anti-parasite and anti-nematode activity, an anthelmintic agent comprising as an active ingredient said substance MBA 090-18. This invention also relates to a feed additive containing a linear peptide compound effective on the improvement of feed effeciency in animal.

Some microorganisms have been known to produce parasiticidal substances, for example, avermectins which belong to macrolide antibiotics and hygromycin B which belongs to destomycin antibiotics. Avermectins are shown by the formula (I) and factors of avermectins are listed in the following Table. In the Table, - represents for a double bond and sugar moiety is α-L-oleandrose in all of the factors.

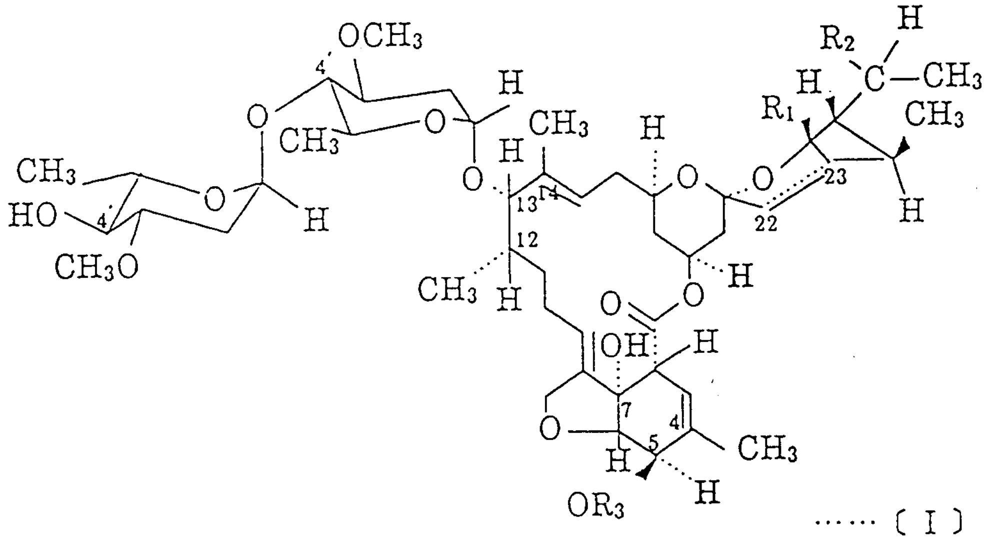

...... ( I )

| Avermectin | $R_1$ | $R_2$ | $R_3$ |
|---|---|---|---|
| $A_1a$ | - | $C_2H_5$ | $CH_3$ |
| $A_1b$ | - | $CH_3$ | $CH_3$ |
| $A_2a$ | OH | $C_2H_5$ | $CH_3$ |
| $A_2b$ | OH | $CH_3$ | $CH_3$ |
| $B_1a$ | - | $C_2H_5$ | H |
| $B_1b$ | - | $CH_3$ | H |
| $B_2a$ | OH | $C_2H_5$ | H |
| $B_2b$ | OH | $CH_3$ | H |

Hygromycin B is shown by the following formula (II).

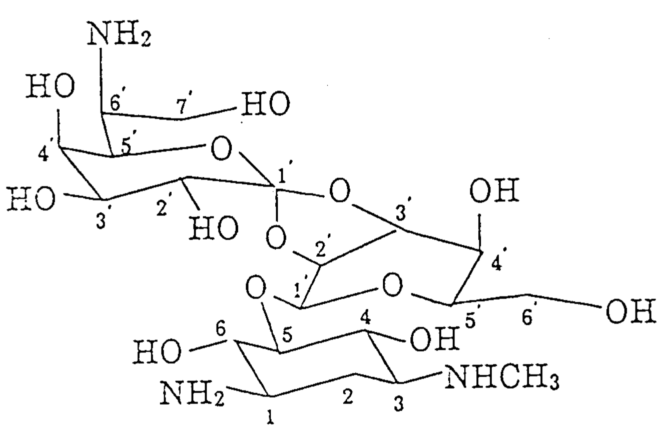

...... ( II )

However, these existing anthelmintic compounds have some drawbacks such as high toxicity and/or poor effects. Therefore, improved and novel anthelmintic or parasiticidal substances continue to be needed.

In the field of stock farming, antibiotics have been routinely administered to animals for the improvement of feed effeciency. The improvement of the feed effeciency leads to the increase in the growth, weight of an animal, which brings said animal to a weight sufficient for marketing in a shorter period of time. Typical antibiotics currently used for such a purpose are polyether antibiotics including monensin and salinomycin, which are known as ionophores and can be shown by the following formulae (III) and (IV), respectively.

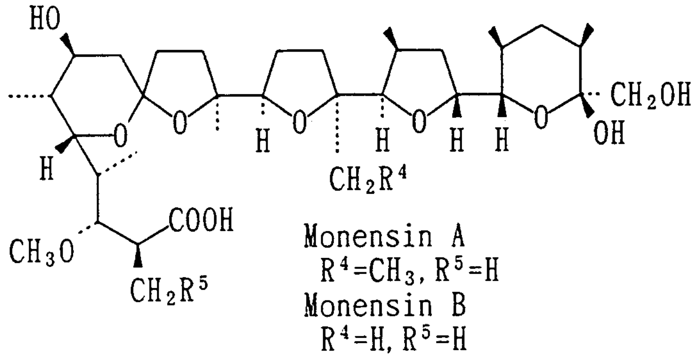

Monensin A
$R^4=CH_3$, $R^5=H$
Monensin B
$R^4=H$, $R^5=H$
Monensin C
$R^4=R^5=CH_3$

...... [III]

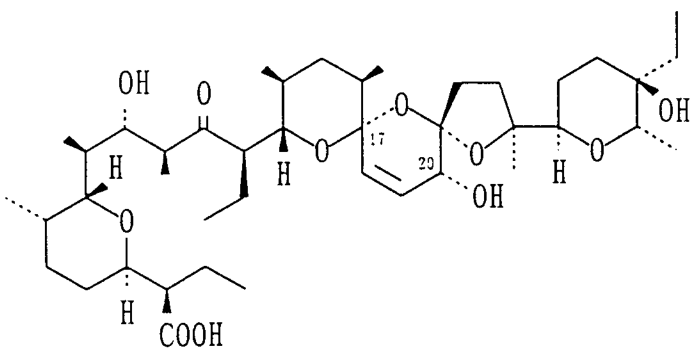

...... [IV]

These polyether antibiotics increase the production ratio of propionic acid which is the energy resource of a ruminant animal and prevent the generation of methane gas which is one of the main causes of energy loss during the rumen fermentation, thereby improving the feed effeciency of the animal. The ionophore effects can be observed broadly among animals having single stomach with well-developed cecum and large intestine. Examples of such animals include pigs, horses, rabbits and the like.

These polyether antibiotics are however usually highly toxic and therefore generally designated as drastic medicines whose administration is strictly restricted. Accordingly, a novel and low-toxic agent having an ionophore activity comparative to that of the polyether antibiotics has been strongly demanded.

Under these circumstances, the present inventors have made investigations with the aim of developing a novel compound which is low-toxic and useful as an active ingredient for parasiticidal agent and/or feed additive for animals. The present inventors have now found that a novel peptide compound isolated from the culture of a strain of genus Verticimonosporium possess the both activities. Actually, said compound was contained in both the culture supernatant and cells.

Thus, the present invention provides a peptide compound isolated from the culture of a strain of genus Verticimonosporium.

The novel peptide compound which is referred to as substance MBA090-18 can be isolated from a strain of Verticimonosporium ellipticum, designated D1528 isolated from fallen leaves and branches by the present inventors.

Verticimonosporium ellipticum D1528 has been deposited with the Fermentation Research Institute, Agency of Industrial Science and Technology, 1-3, Higashi 1 chome, Tsukuba-shi, Ibaraki-ken, Japan, under the deposit No. Ferm P-11747 on the third of October, 1990. Furthermore, the same organism has been

additionally deposited with the same depository under the deposit No. FERM BP-3663 on November 26, 1991 according to the stipulations of the Budapest Treaty.

Among peptide compounds produced by Verticimonosporium ellipticum D1528, substance MBA090-18 having the following physicochemical characteristics proved to be useful as a parasiticidal agent and feed additive of the invention.

Thus, the present invention provides a novel peptide compound MBA090-18 having the following physicochemical properties:

a) Appearance: powder, light brown

b) Optical rotation: $[\alpha]_D = -8.3°$ (c 0.2, methanol, 25°C)

c) Melting point: 192 - 194°C

d) Elemental analysis (found): C, 49.86; H, 6.79; O, 13.63

e) Molecular weight: 1999 - 2001 (the calculated molecular weight 1999 was obtained by assuming the atomic weight of hydrogen atom to 1 and that of other atoms to the double of the atomic number)

f) UV spectrum: no characteristic spectrum can be observed beyond wave length of 220 nm.

g) IR (KBr): 3300, 2990, 2930, 1660, 1540, 1450, 1380, 1300, 1200, 1180, 1140 ($cm^{-1}$)

h) $^1H$ NMR ($CD_3OD$ - TMS; 500 MHz) $\delta$ ppm: 1.01, 1.09, 1.46-1.63, 1.88, 2.10, 1.90-2.40, 2.60, 2.80, 2.96, 3.20-3.40, 3.49, 3.67, 3.68, 3.78, 3.83, 3.92, 3.93-4.29, 4.39, 4.50, 4.62, 7.0-7.4

i) $^{13}C$ NMR ($CD_3OD$ - TMS; 125 Mhz) $\delta$ ppm: 16.7, 16.9, 17.1, 19.4, 20.9, 23.1, 23.2, 23.3, 23.4, 23.5, 23.6, 23.7, 24.1, 24.2, 24.3, 26.3, 26.5, 26.7, 26.9, 27.0, 27.1, 27.2, 27.3, 27.6, 27.7, 29.9, 30.2, 32.5, 32.6, 32.9, 37.9, 39.4, 45.8, 48.3, 50.3, 50.8, 51.6, 53.8, 53.9, 54.2, 57.1, 57.2, 57.3, 57.4, 57.5, 57.6, 57.7, 57.8, 57.9, 58.0, 58.1, 58.2, 59.6, 64.6, 65.1, 65.2, 127.7, 127.8, 129.3, 129.4, 130.4, 130.6, 138.0, 138.5, 172.1, 173.1, 174.1, 175.0, 175.1, 175.4, 175.5, 175.6, 176.6, 176.8, 176.9, 177.0, 177.2, 177.3, 177.4, 177.7, 178.1, 178.2, 178.3, 178.4, 178.5, 178.7, 178.8, 178.9

j) Solubility: soluble in water, methanol and ethanol; and slightly soluble or insoluble in acetone, chloroform and ethyl acetate.

k) Color reaction: Ninhydrin reaction: negative.

Microbiological characteristics of Verticimonosporium ellipticum D1528, which is hereinafter referred to as "D1528", are described below.

(1) Morphological Characteristics:

Colony expands to 3.5 cm in diameter in 2 weeks when plated on the surface of PDA media, which colony is smooth as velvet and the surface and the reverse colors are white and yellowish white, respectively. Substrate and aerial mycelia are fairly well developed, branched and have septal walls. Conidiophores grow from aerial mycelia to 100 - 200 μμm in length and 1.5 - 3.0 μm in width. Conidiophores have septal walls where segmentation and sloughing take place. Conidium-producing (conidiogenous cells) cells grow in verticillation consisting of 3 to 7 cells which are markedly swollen at the base and pointed at the end (8 - 12.6 - (25)x2.6 - 3.6 μm). The conidium is aleuroconidium type; oval in shape, 6 - 8.3x3 - 4 μm in size; colorless; and smooth.

(2) Physiological characteristics:

The microorganism can grow under the following conditions:

Optimum growth conditions:

Ph 5 -7 (in LCA liquid medium for 2 weeks); and temperature 20 - 27°C (on PDA medium for 2 weeks).

Possible growth conditions:

Ph 3 to 9 (in LCA liquid medium for 2 weeks); and temperature 15 - 30°C (on PDA medium for 2 weeks).

The microorganism can not grow at 37°C.

As can be seen from the above, D1528 has the following outstanding characteristics: a) conidiophores have septal walls where segmentation and sloughing take place; b) conidium-producing cells grow in verticillation from a main axis; c) conidium is aleuroconidium type; and d) conidium is oval in shape. The above characteristics of strain D1528 are well agreed with the description of species Verticimonosporium ellipticum given by Matsushima, "Icones Microfungorum a Matsushima Lectorum" Publ. by the Author, pp. 162(1975)). Known species of genus Verticimonosporium are V. ellipticum and V. diffractum which are distinguishable on the basis of the conidium. The conidium of the former is oval and that of the latter globose.

The cultivation of D1528 can be carried out using any of the well known procedures in the art to give the cultured broth in an aqueous nutrient medium containing assimilable sources of carbon, nitrogen, and

inorganic salts.

The nutrient medium may contain carbon source(s) (e.g., glucose, starch syrup, dextrin, sucrose, starch, molasses, animal or vegetable oil and the like) and inorganic or organic nitrogen source(s) (e.g., organic sources such as soybean flour, corn steep liquor, fish meal, yeast extract, meat extract, peptone and inorganic sources such as ammonium salts including ammonium sulfate, ammonium chloride and the like). If desired, other nutritious sources generally used for the improvement of growth of bacteria and the productivity of antibiotics [e.g., inorganic salts, preferably salts of heavy metals, trace metal and/or vitamins (e.g., vitamin $B_1$)] may be added to the medium.

The cultivation of D1528 is usually carried out at pH 6.0 - 9.0 and at 15 - 30 °C, preferably at pH 6.5 - 7.5 and at 20 - 27 °C for about several days under a submerged aerobic fermentation conditions until a sufficient amount of substance MBA090-18 is produced.

The desired substance MBA090-18 can be obtained from the cultured broth conventionally. When the cultivation completes, the cultured broth is subjected to centrifugation or precipitation to separate the culture supernatant and cells. Although substance MBA090-18 is contained in both of the supernatant and the cell pellet, the content is extremely high in the latter.

For the isolation of the product, the pellet is extracted with an appropriate solvent such as acetone and the extract concentrated. After a series of extractions with organic solvents, a crude product is obtained as powder. Thus obtained crude product can be purified using any of conventional procedures such as extraction with a solvent, crystallization, column chromatography, high performance liquid chromatography or the like. If the supernatant is employed for the isolation and purification of substance MBA090-18, it can be treated substantially in the same manner as above.

The present invention also provides the process for producing the substance MBA090-18 by cultivating a strain of genus Verticimonosporium capable of producing substance MBA090-18 in a medium under an appropriate condition until the substance MBA090-18 is produced and isolating said substance from the culture.

The present invention further provides the process for producing the substance MBA090-18 by cultivating Verticimonosporium ellipticum D1528 (FERM BP-3663) in a medium under an appropriate condition until a sufficient amount of substance MBA090-18 is produced and isolating the substance MBA090-18 from the culture.

As can be seen from the Experiments described below, the substance MBA090-18 can suppress or inhibit the growth and motility of nematodes which infect to animals.

Accordingly, the present invention provides an anthelmintic agent comprising as an active ingredient the substance MBA090-18.

The present invention also provides a parasiticidal agent comprising as an active ingredient the substance MBA090-18.

The amount of substance MBA090-18 effective for treating or preventing parasitic diseases of an animal varies depending on the species of the animal and the parasite affecting on the animal. However, it can be between 0.1 mg/day/kg and 100 mg/day/kg, preferably, 1 mg/day/kg and 10 mg/day/kg weight of the animal.

Thus, the present invention provides a method for treating or preventing parasitic diseases of an animal by administering therapeutically effective amount of MBA090-18 to the animal.

For the administration, substance MBA090-18 can be formulated into an appropriate formulation employing carrier(s), diluent(s), filler(s) or the like generally used in the field of veterinary.

The substance MBA090-18 can be conveniently administered orally together with a feed ration or drinking water, or solely. However, other routes of administration can be employed, for example, by implant or by intramuscular or intravenous injection. For oral administration, the substance MBA090-18 is formulated in association with one or more carriers or the like into an appropriate form such as powder, pellet, capsule or the like. Alternatively, it can be administered by mixing into drinking water after formulating into a wettable powder or a concentrated emulsion.

As previously described, the present invention relates to a method for improving the feed effeciency of an animal, which comprises administering a linear peptide compound to the animal.

Thus, the present invention provides a feed additive comprising as an active ingredient a linear peptide compound.

Preferably, the peptide compound has ionophore activity. Especially preferable compounds are substance MBA090-18, gramicidin D, antiamoebin and alamethicin. For the preparation of a feed additive of the invention, a purified peptide compound is not necessarily required, but a crude compound can be used so long as it is physiologically acceptable and has the activity of improving the feed effeciency. For example, if said peptide is a product of a microorganism such as MBA090-18, a biologically pure culture of the

microorganism, e.g., D1528, capable of producing the peptide compound is also useful as an ingredient of feed additive of the invention. Therefore, the cell pellet obtained from culture of D1528 or a roughly processed material thereof are also available.

For the purpose of the improvement of the feed effeciency, a peptide compound can be administered as such. However, as mentioned in the above, it can be formulated into an appropriate formulation in the form of pellet, powder, capsule or the like in association with suitable carriers, fillers, excipients, or diluents and administered orally. Generally, a selected peptide compound can be conveniently administered by just mixing into drinking water or feed ration. When the peptide compound is mixed with feed, it is firstly diluted with an appropriate diluent or filler at a concentration of from 0.1 to 50 % by weight and mixed with feed to give a suitable final concentration. Examples of diluents or fillers include conventional components of feed ration for the animal such as defatted rice bran, soybean cake, corn meal, wheat flour and the like. Additionally, starch, glucose, lactose or sugar can be used.

The final concentration of a peptide compound can be in the range of from about 1 to 1000 ppm in the feed ration and from about 1 to 1000 mg/L in drinking water. In the latter case, the compound may be merely dissolved into water. Alternatively, it is formulated into a wettable powder by conventional method or a concentrated emulsion by suspending into an emulsifier such as a ester of sucrose and aliphatic acid, which is dissolved into water before use.

The daily dose of peptide compound for increasing the feed effeciency varies depending on the peptide selected and the animal to be treated. However, it can be in the range of from 0.05 to 50 mg/day/kg weight of the animal.

Examples of animals to which the present feed additive can be administered include ruminant animals such as cattle, sheep, goat and the like and those having a single stomach such as pig, horse, rabbit and the like. The treatment with the feed additive of the invention can be carried out continuously. However, it should be started after the weaning period, that is, after the development of the rumen in case of ruminant animals and after the development of cecum and large intestine in case of animals having single stomach.

As mentioned in the above, the feed additive of the invention improves the feed effeciency. The term "feed effeciency" herein used refers to the extent of feed intake and consumption (utilization) by animal. The improvement of feed effeciency results in the increase in the weight of animal with smaller amount of feed. As will be described in the following Experiments, the feed additive of the invention exerts the activity through the increase in the production ratio of propionic acid, an energy resource of a ruminant animal, and the reduction of the generation of methane gas, one of causes of energy loss.

Thus, ruminant animals generally uses as energy resource volatile lower fatty acids (hereinafter, referred to as VFA) including acetic acid, butyric acid, propionic acid and the like produced in the rumen. Among them, propionic acid is superior to the rest in terms of energy effeciency. The peptide compound increase the production ratio of propionic acid among VFAs in the rumen of a ruminant animal, thereby increasing the energy effeciency and improving the feed effeciency.

It is also noted that methane gas is produced in the rumen of a ruminant animal and excluded without utilization as energy. The energy loss owing to the production of methane gas is assumed to be about 8 - 10 % of the whole energy taken by a ruminant animal. The peptide compound decreases the production of methane gas, which attribute to the improvement of feed effeciency.

The above-mentioned phenomena concerning to the production of VFAs or methane gas are also observed in other animals having single stomach. Therefore, the feed additive of the invention is expected to be useful in the improvement of feed effeciency in such animals to some extent, not to the same extent as that observed in ruminant animals.

As can be seen from the Experiments, the substance MBA090-18 improves the rumen fermentation effeciency by increasing the production of propionic acid, decreasing the production of methane gas. These facts demonstrate that the substance MBA090-18 possesses the same ionophore activity as that of existing polyether antibiotics. A comparative experiment conducted using the substance MBA090-18 and monensin revealed that the both compounds are almost equivalent as to the ionophore activity. However, monensin suppresses the cellulose digestion, while substance MBA090-18 does not. Thus, MBA090-18 has ionophore activity and improve the feed effeciency of an animal without reverse effects.

In the accompanying Drawings:

Fig. 1 depicts UV spectra of MBA090-18.

Fig. 2 depicts IR spectra of MBA090-18 (KBr disk).

Fig. 3 depicts $^1$H NMR spectra ($CD_3OD$).

Fig. 4 shows $^{13}$C NMR spectra ($CD_3OD$).

The following examples further illustrate the detail of the present invention. The examples are not intended to be limiting to the scope of the invention in any respect and should not so construed.

Example 1

Preparation of Antibiotic MBA090-18

A. Shake Fermentation

For the shake fermentation, a medium (pH 6.0) with the following composition was prepared.

| Ingredient | Amount (%) |
|---|---|
| Corn starch | 2.0 |
| Glucose | 0.5 |
| Soybean oil | 5.0 |
| Pharmamedia | 2.0 |
| Soybean flour | 2.0 |
| Staminol | 0.2 |
| Calcium carbonate | 1.0 |

The medium (80 ml) was poured into 100 Erlenmeyer flasks (volume: 500 ml) and autoclaved at 121 °C for 20 min and inoculated with one scoop of platinum loop of Verticimonosporium ellipticum D1528. The inoculated media were incubated at 27 °C for 7 days on a rotary shaker at 210 rpm.

The cultured broth was centrifuged and the peletted cell containing substance MBA090-18 in larger extent taken.

B. Isolation and Purification of Substance MBA090-18

The cell pellet was suspended in 2 L of acetone, stirred and centrifuged (5 x $10^3$ g, 15 min). The acetone extract was then concentrated to the volume of 1 L under a reduced pressure. The concentrate was washed with ethyl acetate to separate the aqueous layer. Ethyl acetate was removed from the aqueous solution under reduced pressure to give about 0.7 L solution. After adjusting the pH of the solution to 4.0 with 1N HCl, 0.7 L of 1-butanol was mixed thereto. The mixture was shaken to partition into layers. The butanol layer was taken and concentrated under reduced pressure to the volume of 0.35 L until the solution became turbid. To the suspension was gradually added about 10 ml of n-hexane to promote the precipitation and filtrated. The filtrate was distilled under reduced pressure to remove 1-butanol and n-hexane to give about 3 g of crude product as a brown powder.

The powder was dissolved in methanol and subjected to the preparative chromatography by means of high performance liquid chromatography using YMC-TMS column (YAMAMURA Chemicals, Inc., Japan; 20 x 250 mm) developing with methanol/water (3:1) containing 0.1 % trifluoroacetic acid. Fractions containing MBA090-18 substance were pooled and combined. The solution was evaporated to dryness under reduced pressure to give about 1.3 g of MBA090-18 as a powder.

UV, IR, $^1$H NMR, $^{13}$C NMR and FAB-MS spectra of substance MBA090-18 are shown below.

UV spectrum: no characteristic spectrum can be observed beyond wave length of 220 nm as shown in the accompanying Figure 1.

IR (KBr): 3300, 2990, 2930, 1660, 1540, 1450, 1380, 1300, 1200, 1180, 1140 ($cm^{-1}$) (Fig. 2)

$^1$H NMR ($CD_3OD$ - TMS; 500 MHz) $\delta$ ppm: 1.01, 1.09, 1.46-1.63, 1.88, 2.10, 1.90-2.40, 2.60, 2.80, 2.96, 3.20-3.40, 3.49, 3.67, 3.68, 3.78, 3.83, 3.92, 3.93-4.29, 4.39, 4.50, 4.62, 7.0-7.4 (Fig. 3)

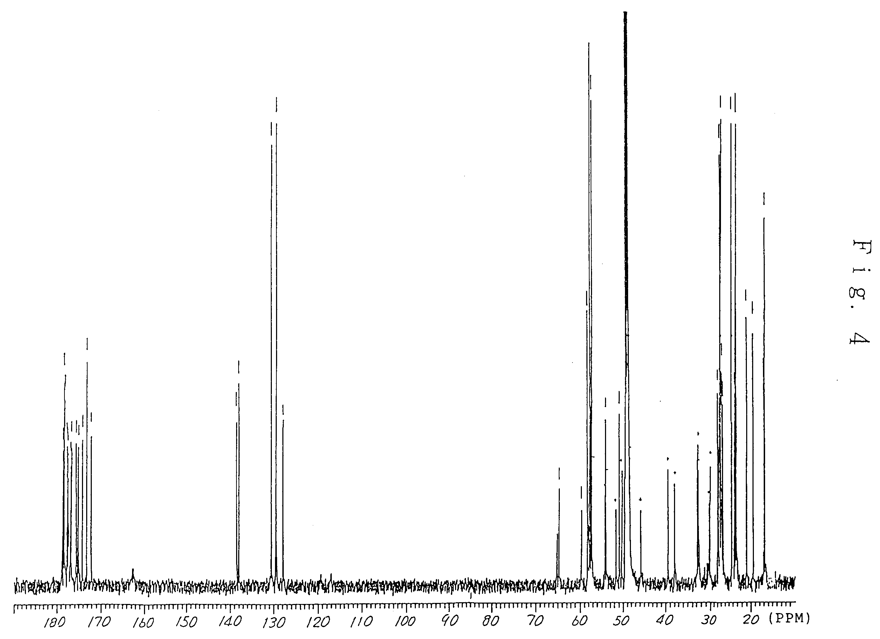

$^{13}$C NMR ($CD_3OD$ - TMS; 125 MHz) $\delta$ ppm: 16.7, 16.9, 17.1, 19.4, 20.9, 23.1, 23.2, 23.3, 23.4, 23.5, 23.6, 23.7, 24.1, 24.2, 24.3, 26.3, 26.5, 26.7, 26.9, 27.0, 27.1, 27.2, 27.3, 27.6, 27.7, 29.9, 30.2, 32.5, 32.6, 32.9, 37.9, 39.4, 45.8, 48.3, 50.3, 50.8, 51.6, 53.8, 53.9, 54.2, 57.1, 57.2, 57.3, 57.4, 57.5, 57.6, 57.7, 57.8, 57.9, 58.0, 58.1, 58.2, 59.6, 64.6, 65.1, 65.2, 127.7, 127.8, 129.3, 129.4, 130.4, 130.6, 138.0, 138.5, 172.1, 173.1, 174.1, 175.0, 175.1, 175.4, 175.5, 175.6, 176.6, 176.8, 176.9, 177.0, 177.2, 177.3, 177.4, 177.7, 178.1, 178.2, 178.3, 178.4, 178.5, 178.7, 178.8, 178.9 (Fig. 4)

FAB-MS spectrum: m/z 2000 $(M + H)^+$
2022 $(M + Na)^+$
2038 $(M + K)^+$

Other physicochemical properties are given below.

Optical rotation: $[\alpha]_D$ = -8.3° (c 0.2, methanol, 25°C)

Melting point: 192 - 194°C

Elemental analysis (found): C, 49.86; H, 6.79; O, 13.63

Molecular weight: 1999 - 2001 (the calculated molecular weight 1999 was obtained by assuming the atomic weight of hydrogen atom to 1 and that of other atoms to the double of the atomic number)

Solubility: soluble in water, methanol and ethanol;

and slightly soluble or insoluble in acetone, chloroform and ethyl acetate.

Color reaction: Ninhydrin reaction: negative.

The following experiments were conducted to demonstrate the antiparasitic and ionophore activities of substance MBA090-18.

Experiment 1

Anti-nematode Activity (1)

Anti-nematode activity of substance MBA090-18 was evaluated using Caenorhabditis elegans, a soil autotrophic nematode.

Caenorhabditis elegans was suspended into sterilized water at the density of 20 - 30 heads/100 µl. To the suspension was added substance MBA090-18 at the concentration of 12.5 ppm. The mixture was incubated at 20 °C for one week while the growth and motility of nematodes being observed. As the positive controls, avermectins (12.5 ppm) and hygromycin B (12.5 ppm) were used, separately. The anti-nematode activity was evaluated and expressed according to the following 5 scales, - ±, +, + + and + + +. Results are shown in Table 1 below.

Table 1

| Test Substance (ppm) | Antinematode Activity | |
|---|---|---|
| | mobility | growth |
| MBA090-18 (12.5) | + + + | + + + |
| Avermectin (12.5) | + + + | + + + |
| Hygromycin B (12.5) | ± | + + |

Experiment 2

Anti-nematode activity (2)

Anti-nematode activity of substance MBA090-18 was evaluated on Trichostrongylus axei which infects to ruminant domestic animals.

Meriones unguiclatus was infected with T. axei and the nematode was subcultured. To each culture was added MBA090-18 at a different concentration and the inhibition of growth by the substance MBA090-18 was observed in vitro. The growth inhibition was observed at the concentration of 50 ppm.

Experiment 3

The acute toxicity of substance MBA090-18 was determined using five male mouse on oral administration. The results are shown in Table 2 below.

Table 2

| | |
|---|---|
| MBA090-18 | ≧ 100 mg/kg |
| Monensin | 43.8 mg/kg |
| Salinomysin | 50 mg/kg |
| Avermectins | 35 mg/kg (*). |

(*): value from a literature

Table 2 shows that the acute toxicity of substance MBA090-18 of the invention is extremely low

compared with that of the commercially available counterparts. Thus, said substance is safer than existing compounds.

Experiment 4

The effect of MBA090-18 on the rumen fermentation was evaluated using a goat ( 20 kg) of a native species of Japan. For the collection of rumen content, a goat was equipped with rumen fistula. The rumen content was collected through the fistula and filtered with gauze. To the 10 ml of filtrate was added 50 ml of a basal medium consisting of the following ingredients, sealed anaerobically under an atmosphere of $CO_2$ gas and incubated at 37 °C for 21 hr. When the incubation finished, the cultured broth was promptly assayed about the production of the total and each of volatile lower fatty acid (VFA). The generation of methane gas and the number of survival protozoas were also determined. The results are given in Table 3 below.

Table 3

| | MBA090-18 | | | | | Monensin |
|---|---|---|---|---|---|---|
| Amount (μg/ml) | 0 | 12.5 | 25 | 50 | 100 | 5 |
| total VFA (mmol/L) | 113 | 105 | 109 | 104 | 89 | 101 |
| each VFA (%) | | | | | | |
| AA | 49 | 53 | 50 | 41 | 44 | 51 |
| PA | 21 | 28 | 32 | 43 | 45 | 28 |
| BA | 23 | 15 | 14 | 14 | 9 | 18 |
| others | 7 | 4 | 4 | 2 | 2 | 3 |
| PA/AA | 0.43 | 0.53 | 0.64 | 1.05 | 1.02 | 0.55 |
| Methane (mmol/L) | 10.1 | 7.5 | 5.1 | 1.5 | 0.3 | 6.5 |
| Protozoa ($x10^4$/ml) | 15 | 11 | 8 | 6 | 6 | 6 |

Abbreviations used are:

AA: acetic acid; PA: propionic acid; and BA:butyric acid.

The basal medium was prepared by mixing the following ingredients, aerated with $CO_2$ and adjusted to pH 6.9 with sodium carbonate.

| Ingredient | Content (g/L) |
|---|---|
| Glucose | 1.0 |
| Cellobiose | 1.0 |
| Maltose | 1.0 |
| Peptone | 2.0 |
| Yeast extract | 1.0 |
| Mineral mixture | 3.0 |

As can be seen from the above Table 3, the production ratio of propionic acid was markedly increased by the addition of substance MBA090-18. The production of methane was decreased. These results indicate that the substance MBA090-18 certainly contribute to improve the feed effeciency.

Ketosis, a typical physiological disease of ruminant domestic animals, is known to be resulted from the increase in the plasma concentration of ketone-body and insufficiency of propionic acid supply. It is also well recognized that the ketosis can be prevented and treated by the administration of propionic acid and/or salts thereof. Therefore the administration of substance MBA090-18 is expected to lead to the increase in the production of propionic acid in the rumen, whereby it is effective on the prevention and treatment of ketosis.

Experiment 5

The effect of various peptide compounds on the rumen fermentation was evaluated substantial in accordance with the procedures described in the above Experiment 4 except that 30 ml of basal medium was used and that the incubation time was 24 hours. The results are given in Table 4 below.

Table 4

| | none | MB | GR | | A L | | AN | |
|---|---|---|---|---|---|---|---|---|
| Amount (μg/ml) | 0 | 15 | 6 | 15 | 6 | 15 | 6 | 15 |
| VFA | | | | | | | | |
| total(mmol/L) | 54.6 | 42.9 | 46.6 | 44.1 | 39.7 | 36.3 | 50.5 | 36.3 |
| each VFA (%) | | | | | | | | |
| AA | 54.0 | 53.8 | 47.9 | 47.8 | 47.6 | 37.7 | 49.9 | 49.6 |
| PA | 21.1 | 33.6 | 32.0 | 32.9 | 29.2 | 43.5 | 26.7 | 26.2 |
| BA | 19.6 | 11.2 | 18.9 | 18.8 | 21.9 | 17.6 | 20.2 | 20.7 |
| others | 5.3 | 1.4 | 1.3 | 0.5 | 1.3 | 1.1 | 3.2 | 3.6 |
| PA/AA | 0.39 | 0.62 | 0.67 | 0.69 | 0.61 | 1.15 | 0.54 | 0.53 |
| Methane (mmol/L) | 9.5 | 8.4 | 7.3 | 6.3 | 9.5 | 1.8 | 10.5 | 10.7 |

Abbreviations used are:

AA: acetic acid; PA: propionic acid; BA:butyric acid; MB; MBA090-18; GR: gramicidin D; AN: antiamoebin; and AL: alamethicin.

Experiment 6

The effect of substance MBA090-18 on the digestion of cellulose in the rumen was evaluated.

A goat (a native species of Japan, 20 Kg) equipped with rumen fistula was again used. A preparation of rumen bacterial solution was prepared by collecting the rumen content through the fistula, followed by a prompt centrifugation at 3000 rpm for 10 min to remove feed debris and protozoa.

To the 10 ml of the bacterial solution was added 50 ml of a basal medium described in the above Experiment 3 except that 0.4 g of cellulose powder and 6 mg of vitamin mixture are supplemented. The flask is then sealed anaerobically under $CO_2$ gas and incubated at 37 °C for 3 days. When the incubation finished, the cultured broth was assayed about the digestion of cellulose. The results are given in Table 5

below.

Table 5

| | MBA090-18 | | | Monensin |
|---|---|---|---|---|
| Amount (μg/ml) | 0 | 12.5 | 25 | 5 |
| Digestion Rate of Cellulose (%) | 72 | 70 | 72 | 10 |

As can be seen from the above Table 4, the cellulose digestion rates observed in animals treated with MBA090-18 are almost the same as that observed in control animal treated with neither of MBA090-18 nor monensin, whereas the digestion rate observed in animal treated with monensin is extremely low. These results indicate that substance MBA090-18 of the invention is safer than monensin.

Experiment 7

The effect of linear peptide compounds on the feed effeciency was evaluated in vivo using a ruminant animal.

A goat (a native species of Japan, 25 Kg) equipped with rumen fistula was used. As a feed additive, either of linear peptide compounds, MBA090-18 and gramicidin D, was administered to the animal. Thus, the animal was given 340 g of a feed (twice a day) containing a test compound at a given concentration shown in the following Table 6. The rumen content was collected through the fistula and subjected to the assay in the same manner as described in the above Experiment 4 to obtain the total amount of VFAs, the production ratio of propionic acid to acetic acid (PA/AA) and the number of survival protozoas. The results are given in the following Table 6. In the table, values are shown as the percentage to the control values (100) obtained by keeping animal with the same feed ration to which neither of test compounds has been added.

Table 6

| | Control | MBA090-18 | Gramicidin D |
|---|---|---|---|
| Amount (mg/day) | 0 | 160 | 80 |
| total VFAs (%) | 100 | 125 | 103 |
| PA/AA (%) | 100 | 116 | 125 |
| Protozoa (%) | 100 | 82 | 86 |

The above Table 6 shows that linear peptide compounds such as MBA090-18 and gramicidin D increase the production ratio of propionic acid and reduce the survival protozoas in vivo, demonstrating that said compounds are useful in the improvement of feed effeciency in animal.

**Claims**

**1.** A peptide compound, substance MBA090-18, which has the following physicochemical properties:

a) Appearance: powder, light brown

b) Optical rotation: $[\alpha]_D$ = -8.3° (c 0.2, methanol, 25°C)

c) Melting point: 192 - 194°C

d) Elemental analysis (found): C, 49.86; H, 6.79; O, 13.63

e) Molecular weight: 1999 - 2001 (the calculated molecular weight 1999 was obtained by assuming the atomic weight of hydrogen atom to 1 and that of other atoms to the double of the atomic number)

f) UV spectrum: no characteristic spectrum can be observed beyond wave length of 220 nm.

g) IR (KBr): 3300, 2990, 2930, 1660, 1540, 1450, 1380, 1300, 1200, 1180, 1140 ($cm^{-1}$)

h) $^1H$ NMR ($CD_3OD$ - TMS; 500 MHz) δ ppm: 1.01, 1.09, 1.46-1.63, 1.88, 2.10, 1.90-2.40, 2.60, 2.80, 2.96, 3.20-3.40, 3.49, 3.67, 3.68, 3.78, 3.83, 3.92, 3.93-4.29, 4.39, 4.50, 4.62, 7.0-7.4

i) $^{13}C$ NMR ($CD_3OD$ - TMS; 125 MHz) δ ppm: 16.7, 16.9, 17.1, 19.4, 20.9, 23.1, 23.2, 23.3, 23.4, 23.5, 23.6, 23.7, 24.1, 24.2, 24.3, 26.3, 26.5, 26.7, 26.9, 27.0, 27.1, 27.2, 27.3, 27.6, 27.7, 29.9, 30.2,

32.5, 32.6, 32.9, 37.9, 39.4, 45.8, 48.3, 50.3, 50.8, 51.6, 53.8, 53.9, 54.2, 57.1, 57.2, 57.3, 57.4, 57.5, 57.6, 57.7, 57.8, 57.9, 58.0, 58.1, 58.2, 59.6, 64.6, 65.1, 65.2, 127.7, 127.8, 129.3, 129.4, 130.4, 130.6, 138.0, 138.5, 172.1, 173.1, 174.1, 175.0, 175.1, 175.4, 175.5, 175.6, 176.6, 176.8, 176.9, 177.0, 177.2, 177.3, 177.4, 177.7, 178.1, 178.2, 178.3, 178.4, 178.5, 178.7, 178.8, 178.9

j) Solubility: soluble in water, methanol and ethanol; and slightly soluble or insoluble in acetone, chloroform and ethyl acetate.

k) Color reaction: Ninhydrin reaction: negative.

**2.** A process for producing the substance MBA090-18 as claimed in Claim 1 which comprises cultivating a strain of genus Verticimonosporium capable of producing substance MBA090-18 in an appropriate culture medium until the substance MBA090-18 is produced and isolating said substance from the culture.

**3.** The process as claimed in Claim 3, wherein the strain of genus Verticimonosporium capable of producing substance MBA090-18 is Verticimonosporium ellipticum D1528 (FERM BP-3663).

**4.** Verticimonosporium ellipticum D1528 (FERM BP-3663 ).

**5.** An anthelmintic agent comprising as an active ingredient the substance MBA090-18 as claimed in Claim 1.

**6.** The anthelmintic agent as claimed in Claim 5 which has anti-parasite activity.

**7.** The anthelmintic agent as claimed in Claim 5 which has anti-nematode activity.

**8.** A feed additive comprising as an active ingredient a linear peptide compound associated with one or more carriers therefor.

**9.** The feed additive as claimed in Claim 8, wherein the peptide compound has ionophore activity.

**10.** The feed additive as claimed in Claim 8 which is useful for the improvement of the feed effeciency.

**11.** The feed additive as claimed in Claim 10, wherein the improvement of the feed effeciency is attributable to the increase in the production ratio of propionic acid in stomach.

**12.** The feed additive as claimed in Claim 10, wherein the improvement of the feed effeciency is attributable to the reduction of the generation of methane gas in stomach.

**13.** The feed additive as claimed in 8, wherein the peptide compound is selected from a group consisting of substance MBA090-18, gramicidin D, antiamoebin and alamethicin.

**14.** A method for treating or preventing parasitic disease of an animal which comprises administering a therapeutically effective amount of the substance MBA090-18 as claimed in Claim 1 to the animal.

**15.** A method for improving the feed effeciency of an animal which comprises administering a physiologically effective amount of the substance MBA090-18 as claimed in Claim 1 to the animal.

Fig. 1

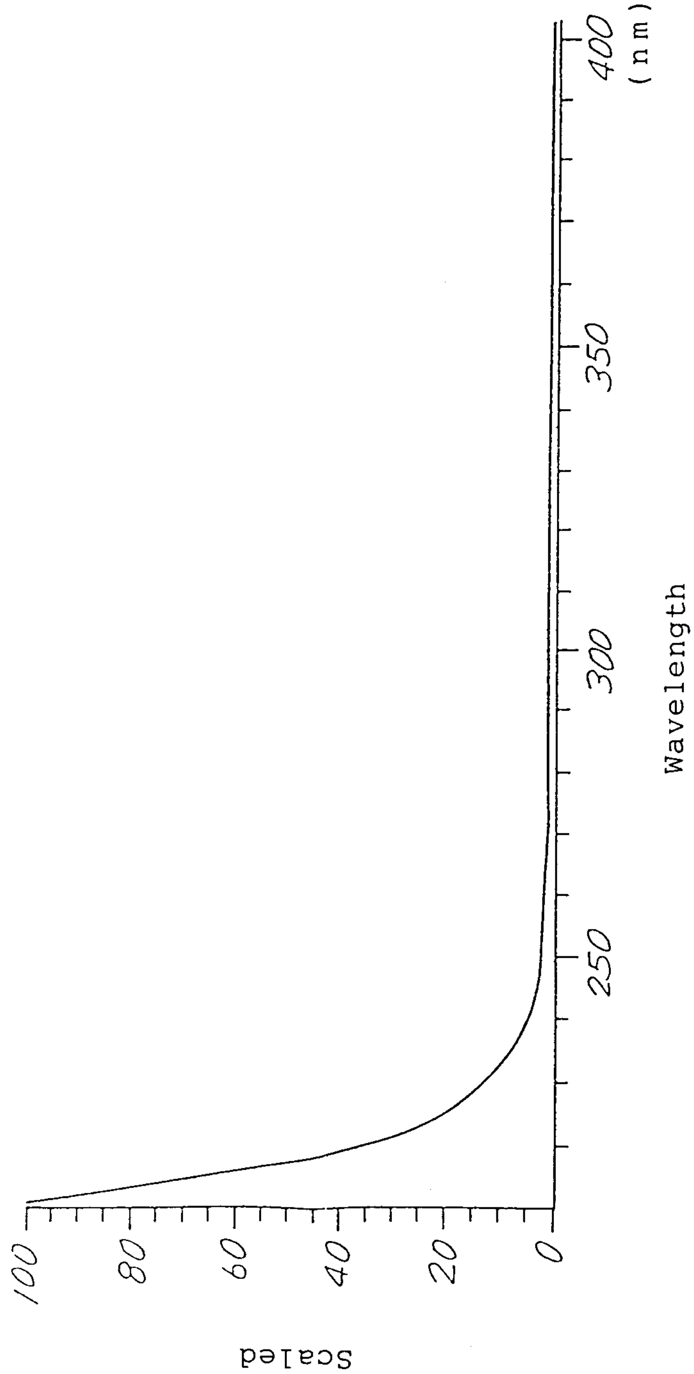

Fig. 2

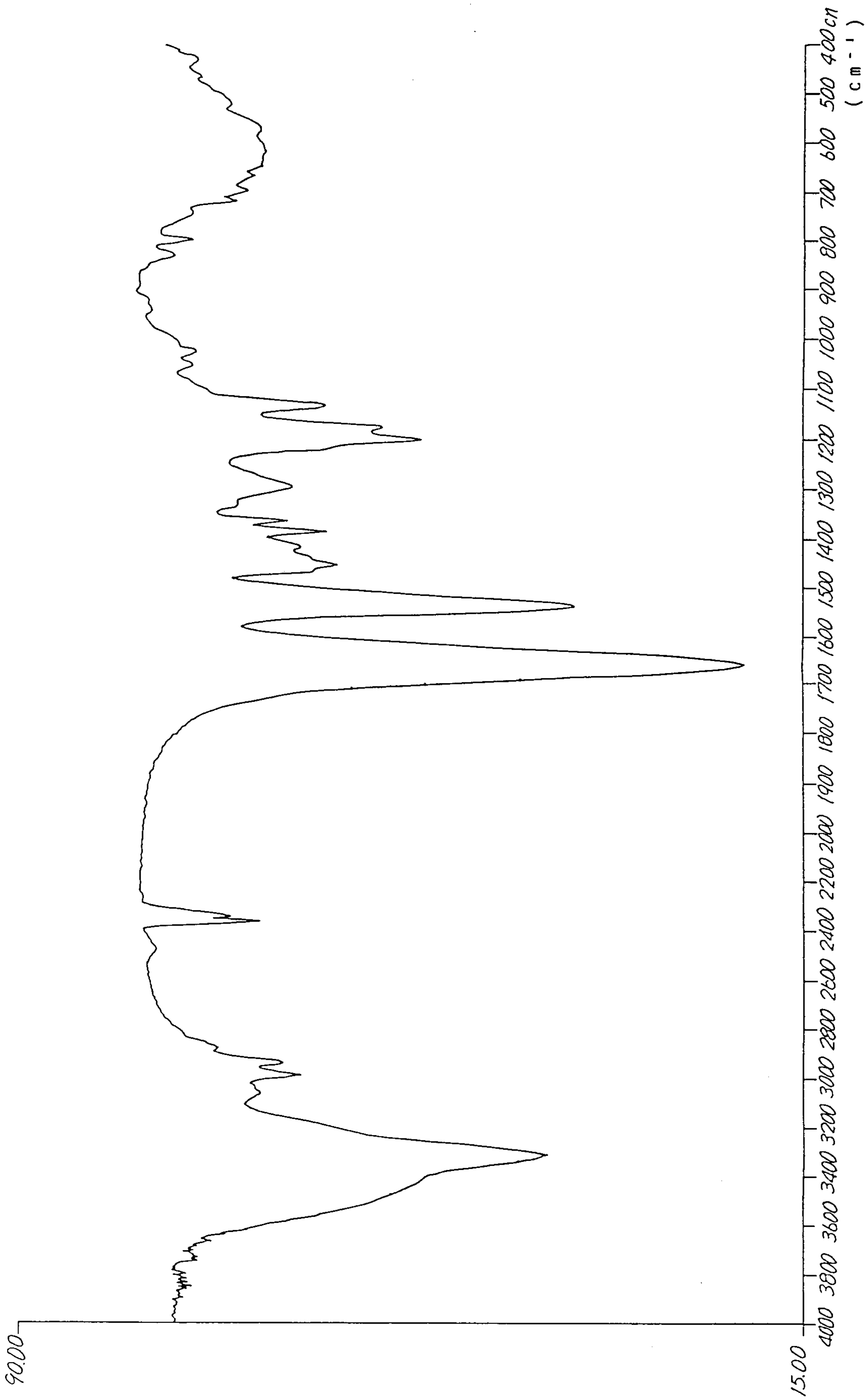
90.00
15.00
4000 3800 3600 3400 3200 3000 2800 2600 2400 2200 2000 1900 1800 1700 1600 1500 1400 1300 1200 1100 1000 900 800 700 600 500 400cm
(cm⁻¹)

F i g. 3

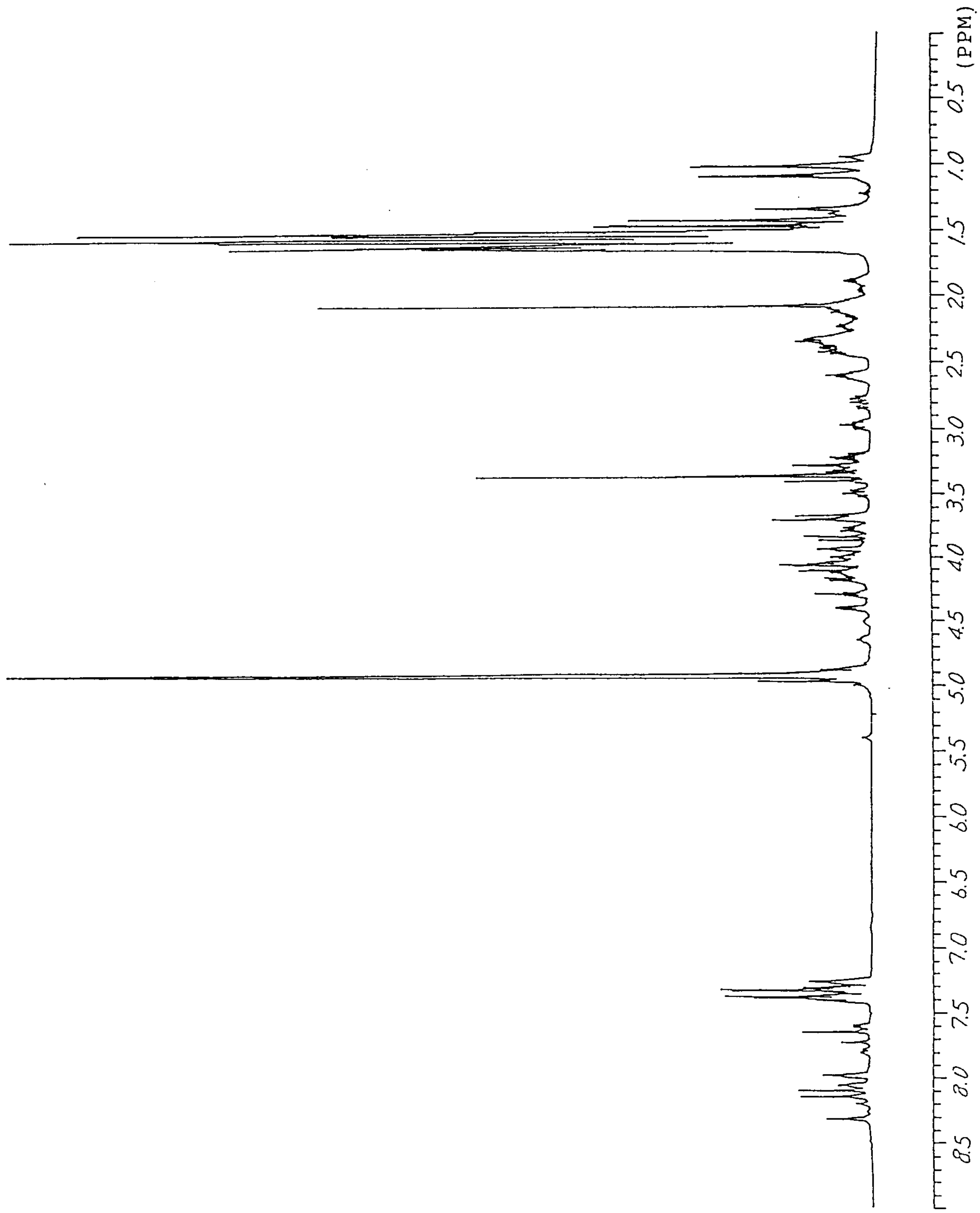

Fig. 4